# EUROPEAN PATENT APPLICATION

(11) **EP 4 591 900 A1**
(43) Date of publication of application: **30.07.2025**
(21) Application number: 25153382.4
(22) Date of filing: 22.01.2025
(51) Int. Cl.: A61M 5/165, A61M 5/38

(54) **FLUID DELIVERY FILTERS**

(30) Priority: 26.01.2024 US 202463625721 P; 13.01.2025 US 202519018981
(71) Applicant: Medtronic MiniMed, Inc., Northridge, CA 91325 (US)
(72) Inventor: Tong, Davy T., Northridge, 91325 (US); Fusselman, Hsi C., Northridge, 91325 (US); Russell, Madeleine D., Northridge, 91325 (US); Chattaraj, Sarnath, Northridge, 91325 (US); Zhang, Guangping, Northridge, 91325 (US); Dang, Kiem H., Northridge, 91325 (US)
(74) Representative: Zimmermann & Partner Patentanwälte mbB

(57) **Abstract**

Filters for use with fluid infusion devices are disclosed herein. According to some embodiments, the present technology includes a system for delivering a fluid medication to a patient at an infusion site, the system comprising a fluid conduit configured to transport a fluid medication along at least a portion of a fluid path extending between a reservoir containing the fluid medication and a cannula subcutaneously inserted in a patient at an infusion site, and a filter disposed within the fluid conduit and configured to trap particulates that form in the fluid medication, wherein the filter comprises a porous structure including glycerin.

## Description

### CROSS-REFERENCE TO RELATED APPLICATIONS

This application claims the benefit of and priority to U.S. Provisional Patent Application Serial No. 63/625,721 filed January 26, 2024, the entire disclosure of which is incorporated by reference herein.

### TECHNICAL FIELD

The present technology relates generally to fluid infusion devices for delivering a medication fluid to the body of a user.

### BACKGROUND

Certain diseases or conditions may be treated, according to modern medical techniques, by delivering a medication fluid or other substance to the body of a patient, either in a continuous manner or at particular times or time intervals within an overall time period. For example, diabetes is commonly treated by delivering defined amounts of insulin to the patient at appropriate times. Some common modes of providing insulin therapy to a patient include delivery of insulin through manually operated syringes and insulin pens. Other modern systems employ programmable fluid infusion devices (e.g., continuous insulin infusion devices such as insulin pumps) to deliver controlled amounts of insulin or other drugs to a patient.

A fluid infusion device suitable for use as an insulin pump may be realized as an external device or an implantable device, which is surgically implanted into the body of the patient. External fluid infusion devices include devices designed for use in a generally stationary location (for example, in a hospital or clinic), and devices configured for ambulatory or portable use (to be carried by a patient). External fluid infusion devices may establish a fluid flow path from a fluid reservoir to the patient via, for example, a suitable hollow tubing. The hollow tubing may be connected to a hollow fluid delivery needle or cannula that is designed to pierce the patient's skin to deliver an infusion fluid to the body.

Persistent problems associated with systems designed to infuse medication include having components that can be difficult for some patients to use, and also that the human body spontaneously reacts against foreign bodies which are introduced into the body, such as an implanted cannula. Among the various responses of a body to foreign bodies, inflammation and the build-up of fibrous tissue at the infusion site significantly shortens the duration that an infusion set may be maintained at a single infusion site (i.e. "site-loss"). Moreover, tissue encapsulation and blockage of the implanted cannula or catheter (i.e. "occlusion") often occurs, thereby impeding or halting infusion of medication. Inflammation may be accelerated due to agglomerations that develop in the insulin as the insulin is stored in a reservoir at ambient temperature within the pump. Thus, frequent re-positioning of the infusion site for continued usage of the infusion pump is required. Currently, wear times of all the commercial insulin infusion sets are labeled for < 3 days.

Accordingly, there is a need for improved fluid delivery systems.

### SUMMARY

Filters for use with fluid infusion devices are disclosed herein. According to some embodiments, the present technology includes a system for delivering a fluid medication to a patient at an infusion site, the system comprising a fluid conduit configured to transport a fluid medication along at least a portion of a fluid path extending between a reservoir containing the fluid medication and a cannula subcutaneously inserted in a patient at an infusion site, and a filter disposed within the fluid conduit and configured to trap particulates that form in the fluid medication, wherein the filter comprises a porous structure including glycerin and/or can have an absorption rate between 0 and 30 seconds, in some cases less than six seconds, or less than five seconds. In several embodiments, the fluid medication is insulin, and the filter is configured to trap insulin aggregates that form in the insulin. The system can be configured to infuse the fluid medication to the patient at the infusion site over a period of time greater than four days, greater than five days, greater than six days, or greater than seven days. In some examples, the porous structure comprises polyvinyl alcohol, a cellulose, a polyurethane, a polyester, a polyether, or a collagen, and has an average pore size of from about 0.1 mm to about 0.5 mm. According to several cases, the porous structure comprises at least one of an acrylic copolymer membrane, a polyethersulfone membrane, a mixed cellulose esters membrane, a cellulose acetate membrane, a cellulose nitrate membrane, a nylon membrane, a hydrophilic polytetrafluoroethylene (PTFE) membrane, and/or a polycarbonate membrane, and has an average pore size of from about 0.1 µm to about 10 µm. In some embodiments, the fluid conduit comprises a tubing and a connector configured to fluidly couple the tubing to the reservoir, and the filter is disposed within the connector. According to several examples, the system includes a second filter disposed within the fluid conduit.

A system for delivering a fluid medication to a patient at an infusion site, the system comprising a fluid conduit having a first end configured to be fluidly coupled to a reservoir containing the fluid medication and a second end configured to be fluidly coupled to a cannula. The fluid conduit can be configured to transport the fluid medication from the reservoir to the patient through the cannula. The system can further comprise a connector configured to fluidly couple the first end of the fluid conduit to the reservoir and a filter disposed within the connector and configured to trap particulates that form in the fluid medication. The filter can comprise a porous structure including glycerin, and/or can have an absorption rate between 0 and 30 seconds, in some cases less than six seconds, or less than five seconds. In several embodiments, the fluid medication is insulin, and the filter is configured to trap insulin aggregates that form in the insulin. The system can be configured to infuse the fluid medication to the patient at the infusion site over a period of time greater than four days, greater than five days, greater than six days, or greater than seven days. In some examples, the porous structure comprises polyvinyl alcohol, a cellulose, a polyurethane, a polyester, a polyether, or a collagen, and has an average pore size of from about 0.1 mm to about 0.5 mm. According to several cases, the porous structure comprises at least one of an acrylic copolymer membrane, a polyethersulfone membrane, a mixed cellulose esters membrane, a cellulose acetate membrane, a cellulose nitrate membrane, a nylon membrane, a hydrophilic polytetrafluoroethylene (PTFE) membrane, and/or a polycarbonate membrane, and has an average pore size of from about 0.1 µm to about 10 µm. In some embodiments, the fluid conduit comprises a tubing. According to several examples, the system includes a second filter disposed within the fluid conduit.

A system for delivering a fluid medication to a patient at an infusion site, the system comprising an infusion pump, a reservoir configured to store the fluid medication and configured to be received by the infusion pump, a cannula configured for subcutaneous insertion into a tissue of the patient at the infusion site, and a fluid conduit having a first end configured to be fluidly coupled to the reservoir and a second end configured to be fluidly coupled to the cannula. The fluid conduit can be configured to transport the fluid medication from the reservoir to the patient. The system can further include a connector configured to fluidly couple the first end of the fluid conduit to the reservoir, and one or more filters disposed in at least one of the reservoir, the fluid conduit, or the connector. Each of the one or more filters is configured to trap particulates that form in the fluid medication. The one or more filters can comprise a porous structure including glycerin, and/or can have an absorption rate between 0 and 30 seconds, in some cases less than six seconds, or less than five seconds. In several embodiments, the fluid medication is insulin, and the filter is configured to trap insulin aggregates that form in the insulin. The system can be configured to infuse the fluid medication to the patient at the infusion site over a period of time greater than four days, greater than five days, greater than six days, or greater than seven days. In some examples, the porous structure comprises polyvinyl alcohol, a cellulose, a polyurethane, a polyester, a polyether, or a collagen, and has an average pore size of from about 0.1 mm to about 0.5 mm. According to several cases, the porous structure comprises at least one of an acrylic copolymer membrane, a polyethersulfone membrane, a mixed cellulose esters membrane, a cellulose acetate membrane, a cellulose nitrate membrane, a nylon membrane, a hydrophilic polytetrafluoroethylene (PTFE) membrane, and/or a polycarbonate membrane, and has an average pore size of from about 0.1 µm to about 10 µm. According to several examples, the system includes a second filter disposed in at least one of the reservoir, the fluid conduit, or the connector.

Further disclosed herein are filters for use with fluid infusion devices. According to some embodiments, the present technology includes a system for delivering a fluid medication to a patient at an infusion site, the system comprising a fluid conduit configured to transport a fluid medication along at least a portion of a fluid path extending between a reservoir containing the fluid medication and a cannula subcutaneously inserted in a patient at an infusion site, and a filter disposed within the fluid conduit and configured to trap particulates that form in the fluid medication, wherein the filter comprises a porous structure including glycerin.

### BRIEF DESCRIPTION OF THE DRAWINGS

Many aspects of the present disclosure can be better understood with reference to the following drawings. The components in the drawings are not necessarily to scale. Instead, emphasis is placed on illustrating clearly the principles of the present disclosure.
FIG. 1 is a schematic representation of a fluid delivery system in accordance with several embodiments of the present technology.
FIG. 2 is a plan view of a fluid delivery system that includes a fluid infusion device and an infusion assembly in accordance with several embodiments of the present technology.
FIG. 3A is an exploded view of the connector shown in FIG. 2, and FIG. 3B is an assembled view of the connector shown in FIGS. 2 and 3A.
FIG. 4A is an exploded view of another connector for use with the fluid delivery systems of the present technology, and FIG. 4B is an assembled view of the connector shown in FIG. 4A.
FIG. 5 is a perspective view of a fluid delivery system that includes a fluid infusion device configured to be adhered to the skin of the user in accordance with several embodiments of the present technology.
FIG. 6 is a graph showing survival curves for fluid delivery systems utilizing different filter materials.
FIG. 7 is a table showing survival rates for fluid delivery systems utilizing different filter materials.

### DETAILED DESCRIPTION

The subject matter described herein relates to certain assemblies, components, and features of a fluid infusion system configured to treat a medical condition of a patient. The fluid infusion system is used for infusing a fluid medication into the body of a user. The non-limiting examples described below relate to a medical device used to treat diabetes (more specifically, an insulin pump), although embodiments of the disclosed subject matter are not so limited. Accordingly, the fluid medication is insulin in certain embodiments. In other embodiments, however, fluid medications other than insulin may be administered through the described fluid infusion systems such as, for example, drugs to treat pulmonary hypertension, iron chelation drugs, pain medications, anti-cancer treatments, medications, vitamins, hormones, or the like. Moreover, the filters of the present technology can be used with fluid delivery systems other than those described herein.

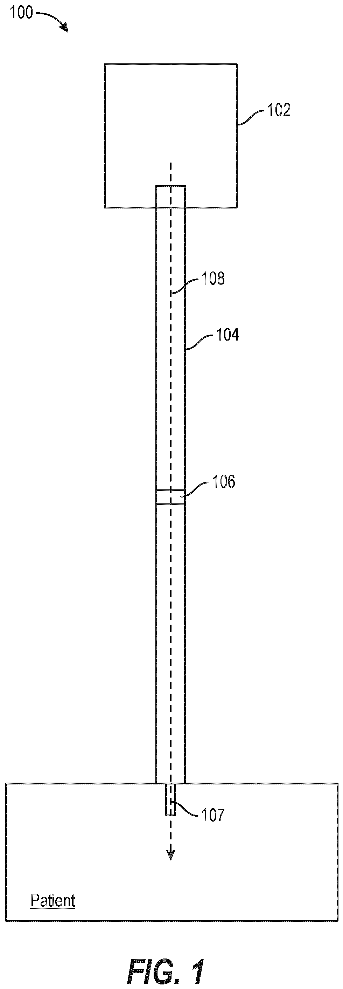

FIG. 1 is a schematic representation of a fluid delivery system 100 configured to administer a fluid medication (such as insulin) to a patient. The fluid delivery system 100 includes a reservoir 102 configured to contain and supply the fluid medication and a fluid conduit assembly 104 configured to be fluidly coupled to the reservoir 102 and convey the fluid medication from the reservoir 102 to the patient along a fluid path 108. The fluid path 108 may terminate at a cannula 107 configured to be implanted within a subcutaneous region of the patient for an extended period of time, such as for at least four days. As detailed herein, the fluid conduit assembly 104 can comprise a structure or series of structures that define all or a portion of the fluid path 108. In some examples, the fluid conduit assembly 104 may comprise tubing and one or more connectors (not depicted) for fluidly coupling the ends of the tubing to the reservoir 102 and cannula 107. The fluid delivery system 100 further includes at least one filter 106 disposed at one or more locations along the fluid path 108 (e.g., disposed within a downstream portion of the fluid reservoir 102, along the fluid conduit assembly 104, or both). As described below, the filter 106 is configured to filter out unwanted particulates present in the fluid medication as it flows along the fluid path 108, thereby reducing the potential for inflammation at the infusion site and advantageously prolonging the duration of cannula implantation. The filter 106 is also configured to enable efficient transmission of the fluid medication through the filter 106, thereby providing improved delivery volume accuracy of the fluid medication.

The filter 106 comprises a porous structure defining a plurality of interconnected pores through which the fluid medication flows during a fluid delivery operation. The average pore size can be small enough to inhibit the flow of particulates, such as insulin aggregates, silicone oil, and other foreign matter. The filter 106 can have an average pore size, for example, of about 0.1 mm to about 5.0 mm, about 0.3 mm to about 1 mm, or no greater than about 0.5 mm. In some embodiments, the filter 106 can have a smaller pore size, such as of from about 1 µm to about 5 µm, or no greater than about 3 µm. In some embodiments, the filter 106 has a porosity of 50% to 95%, and in some cases a porosity of 90% to 95%. In some examples, the filter 106 can comprise a material that expands when in contact with a liquid, such as a foam.

The filter 106 and/or porous structure can be formed of a material or materials that are sufficiently hydrophilic to absorb the fluid medication and expand at a rate that avoids occlusion of the fluid path 108 at the site of the filter 106. For example, the filter 106 can have an absorption rate of between 0 and 30 seconds, and in some cases between 0 and 5 seconds, or less than 5 seconds. The absorption rate can be measured by dropping an unused filter 106 into water and measuring the time it takes for the filter to reach full expansion.

In some embodiments, the porous structure is formed of a first hydrophilic material, which forms the majority of the porous structure, and a second or additive hydrophilic material that is more hydrophilic than the first material. The first hydrophilic material can comprise one or more of polyvinyl alcohol (PVA), polyurethane, polyester, polyether, polyacrylate, nylon, cellulose, cellulose acetate, cellulose nitrate, polyethylene, polyvinyl acetate, polysulfone, polyethersulfone (PES), collagen, polycaprolactone, an acrylic copolymer, mixed cellulose esters, polytetrafluoroethylene (PTFE), polycarbonate, and other hydrophilic materials. In some embodiments, the first hydrophilic material comprises a polymer having a hydrophobic backbone and hydrophilic functional groups (such as PVA). The hydrophobic backbone can attract silicone oil, which may be present in a fluid reservoir. In any case, when combined with the first hydrophilic material, the additive hydrophilic material increases the speed of water absorption and the speed at which the filter expands when in contact with water. Without being bound by theory, it is believed that the increased hydrophilic nature of the filter 106 when the additive hydrophilic material is present improves the filter's ability to filter out unwanted particulates, such as insulin aggregates and others, thereby improving the *in vivo* survivability of the cannula 107. Moreover, improved wettability of the filter 106 can reduce or eliminate pulsatile flow through the filter 106, which not only increases the fluid delivery volume accuracy, but also requires less power from the pumping mechanism of the infusion device.

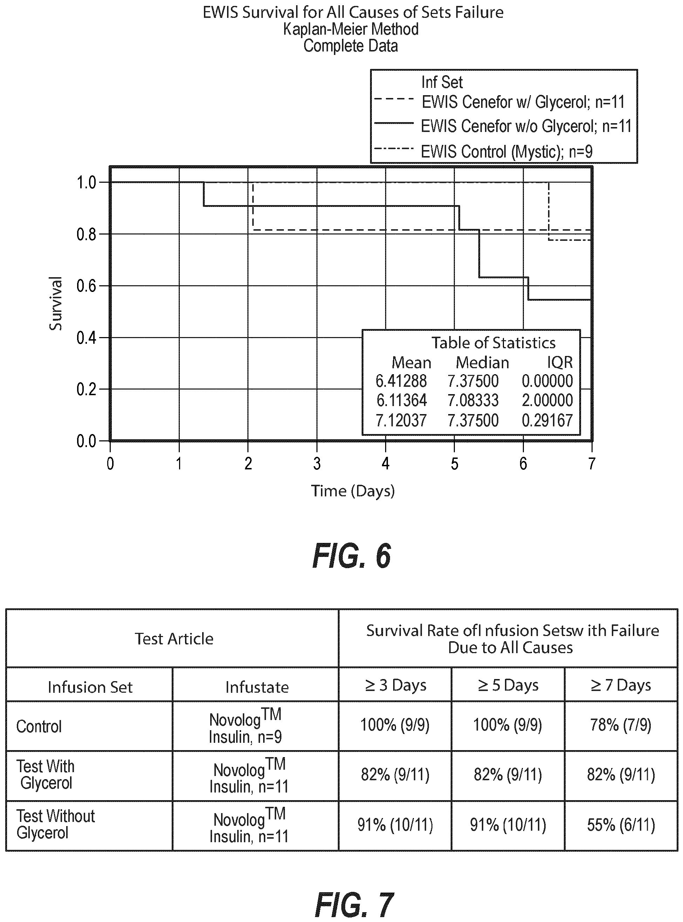

In several embodiments, the second hydrophilic material can comprise glycerol (also referred to as "glycerin"). In addition to being very hydrophilic, glycerol is naturally occurring in the human body and, beneficially, also found in current on-market insulin formulations. The filter 106 can include, for example, of from about 0.01 mg to about 50 mg of glycerol, or about 1 mg to about 25 mg, or about 0.01 mg to about 10 mg. Experimental data showing the improved duration of wear of a transcutaneous delivery system as a result of a glycerol additive in the filter is described below in the Example, and with reference to FIGS. 6 and 7. Other second hydrophilic materials include humectants such as abutanediol, erythritol, sorbitol, mannitol, and others. In any case, the porous structure can include a first hydrophilic material to second hydrophilic material ratio of about 10:1 to about 1:1, or about 5:1 to about 1:1. As one of several example combinations, provided solely for the purpose of providing an example, the porous structure may comprise a PVA (first hydrophilic material) to glycerol (second hydrophilic material) ratio of 10:1 to 1:1, or about 5:1 to 1:1. If the PVA to glycerol ratio is 1:1 and glycerol is 0.01 mg, PVA will be 0.01 mg; if the PVA to glycerol ratio is 10:1 and glycerol is 0.01 mg, PVA will be 0.1 mg; if the PVA to glycerol ratio 1:1 and glycerol is 50 mg, PVA will be 50 mg; if the PVA to glycerol ratio is 10:1 and glycerol is 50 mg, PVA will be 500 mg, etc.

The filter 106 can have a height measured along the axis of the fluid path, a width measured perpendicular to the height, and a volume. In some cases, the filter 106 can have a height of from about 0.05 inches to about 0.2 inches.

Although FIG. 1 shows a single component that serves as the filter 106, the fluid delivery systems 100 of the present technology can utilize a plurality of physically distinct elements that collectively function as the filter 106. As previously mentioned, the fluid delivery system 100 can include multiple filters 106 positioned along the fluid path 108. The filter 106 can be positioned, for example, at one, some, or all of the following locations: within a portion of the reservoir 102; within a tubing extending between the reservoir 102 and the cannula 107; within a connector configured to fluidly couple an upstream end of the tubing to the reservoir 102; within a connector configured to fluidly couple a downstream end of the tubing to the cannula 107. One or multiple filters 106 can be disposed at any of the foregoing locations.

When multiple filters 106 are used, the filters 106 can have the same or different material properties. For example, in some embodiments the fluid delivery system 100 can include first and second filters. The first filter can comprise a first hydrophilic material and a hydrophilic additive (such as glycerol) and have a first pore size and a first height. The second filter can include a second hydrophilic material different than the first hydrophilic material, and a hydrophilic additive (such as glycerol), and have a second pore size smaller than the first pore size and a second height less than the first height.

In some embodiments, the filter 106 also serves to absorb and/or adsorb certain substances, chemicals, or suspended elements from the fluid medication. For example, the filter 106 may include material that is configured or treated to absorb/adsorb lubricating or manufacturing oil that is associated with the manufacturing, assembly, or maintenance of one or more components of the fluid delivery system. In this regard, a fluid reservoir for insulin can be fabricated with a trace amount of silicone oil that serves as a lubricant for the plunger of the reservoir. Accordingly, the filter 106 can include a material, layer, or treatment that reduces, traps, or otherwise removes some or all of the silicone oil from the medication fluid as it passes through the filter 106.

In particular embodiments, the filter 106 optionally serves as a drug depot during operation of the fluid delivery system. To this end, the filter 106 can include a drug, medicine, chemical, or composition impregnated therein (or coated thereon, or otherwise carried by the filter 106). A quantity of the drug is released into the fluid medication as the fluid flows through the filter 106 during a fluid delivery operation. In practice, the drug carried by the filter 106 will eventually be depleted unless the filter 106 or the fluid conduit assembly 104 is replaced before depletion. The drug carried by the filter 106 can be selected to address the needs of the particular patient, fluid delivery system, fluid medication, etc. In accordance with the exemplary insulin infusion system described here, the filter 106 can optionally be impregnated with a drug that treats the infusion site to extend the useful life of the fluid infusion set. For example, the filter 106 can be treated with an anticoagulant such as heparin or dextran. As another example, the filter 106 can be impregnated or infused with an anti-proliferative drug such as rapamycin. It should be appreciated that these examples are neither exhaustive nor restrictive, and that the filter 106 can be impregnated, treated, or infused with any drug that may be appropriate and suitable for the particular medical condition, fluid delivery system, or application.

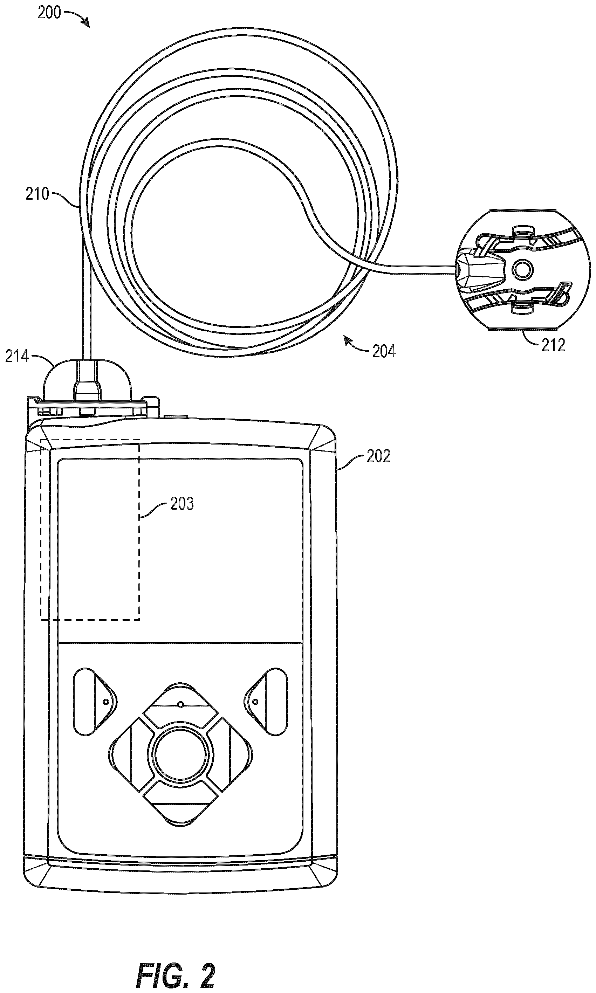

FIG. 2 is a plan view of an example embodiment of a fluid delivery system 200 that includes a portable fluid infusion device 202 for controlled dispensation of a medication fluid and an infusion assembly 204 configured to be fluidly coupled to the infusion device 202. The infusion device 202 is configured to be carried or worn by the patient and includes a fluid reservoir 203 (hidden from view in FIG. 2) containing the fluid medication. The fluid reservoir 203 may be removable from or fixed to the housing of the infusion device 202. The infusion device 202 can also include a plunger slidably disposed within the fluid reservoir, as well as an associated drive system for moving the plunger to dispense the fluid medication from the fluid reservoir 203 into the infusion assembly 204. The infusion device 202 can also include a power source and one or more electronic components (e.g., a processor, a transmitter, etc.).

As depicted in FIG. 2, the infusion assembly 204 includes a tube 210, an infusion unit 212 fluidly coupled to the distal (i.e., downstream) end of the tube 210, and a connector 214 configured to be fluidly coupled to the proximal (i.e., upstream) end of the tube 210. The infusion unit 212 is configured to be temporarily adhered to the patient's body (e.g., via an adhesive patch) and includes a cannula (not shown) configured to be implanted within a subcutaneous region of a patient for an extended period of time (e.g., more than four days). The connector 214 mates with and couples to a portion of the fluid reservoir 203 and/or infusion device housing, thereby establishing the fluid path from the fluid reservoir 203 to the tube 210 and securing the infusion assembly 204 to the infusion device 202. Thereafter, actuation of the drive system of the fluid infusion device 202 causes the fluid medication to be expelled from the fluid reservoir 203, through the connector 214, through the tube 210, and into the body of the patient via the cannula of the infusion unit 212.

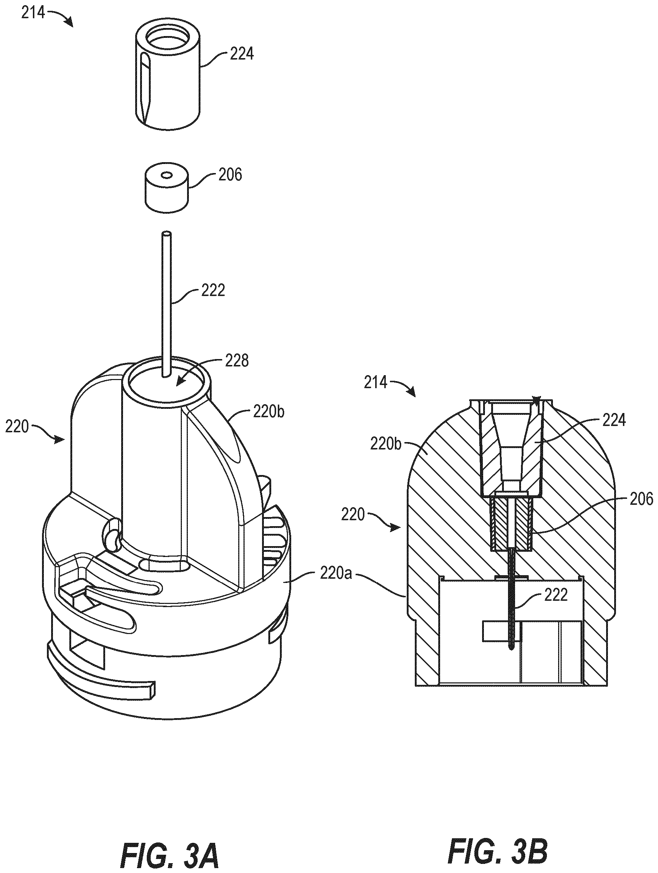

According to several examples, the connector 214 comprises a removable reservoir cap and/or fitting that is suitably sized and configured to accommodate replacement of fluid reservoirs (which are typically disposable) as needed. FIGS. 3A and 3B show exploded and assembled views of the connector 214, respectively. As shown in FIGS. 3A and 3B, the connector 214 includes a body 220 comprising a distal (upstream) portion 220a configured to receive a mating portion of the fluid reservoir 203 (e.g., via a threaded engagement, a snap fit, tabs, etc.), and a proximal (downstream) portion 220b defining a channel 228 and configured to receive an upstream end of the tubing 210. The connector 214 may further include a coupling element 224 configured to be received in the channel 228, a needle 222 having an upstream end configured to penetrate a septum of the fluid reservoir 203, and a filter 206. The filter 206 is positioned within the channel 228 with the first and second filters 206a, 206b upstream of the third filter 206c. The filter 206 can comprise a porous structure formed of a hydrophilic material (such as any of the hydrophilic materials disclosed herein) and a hydrophilic additive, such as glycerol, as detailed above.

As best shown in FIG. 3B, the needle 222 can be secured within the body 220 such that an upstream end of the needle 222 is positioned within an interior cavity of the lower portion 220a, and a downstream end of the needle 222 is positioned within the channel 228, upstream of or aligned with an upstream end of the filter 206. During a fluid delivery operation, the fluid medication is forced out of the fluid reservoir 203 into the hollow needle 222, then through the first filter 206 before flowing into the upstream end of the tubing 210 coupled to the upper portion 220b of the body 220.

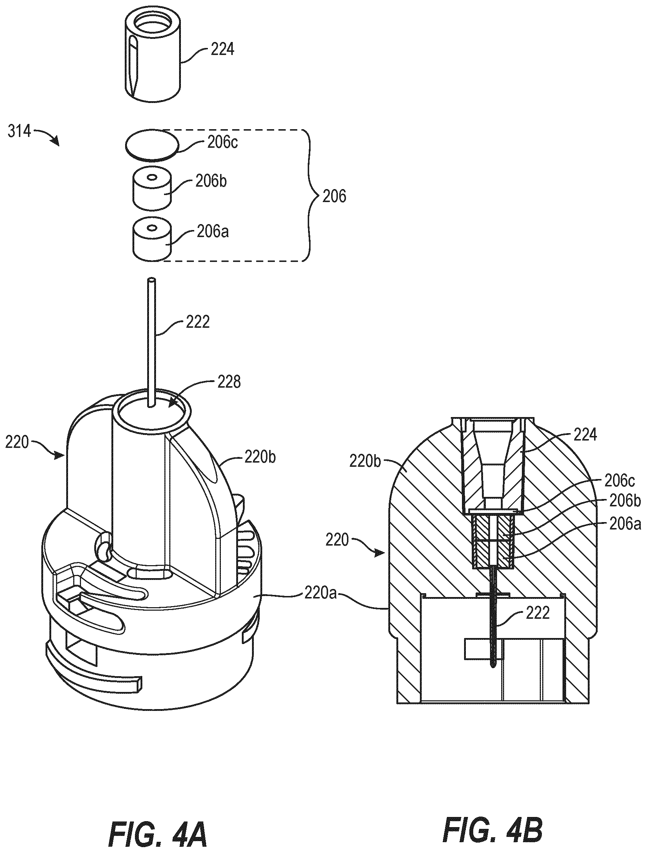

FIG. 4A is an exploded view of another connector 314 for use with the fluid delivery systems of the present technology, and FIG. 4B is an assembled view of the connector shown in FIG. 4A. As shown in FIGS. 4A and 4B, the connector 314 includes a body 220 comprising a lower (upstream) portion 220a configured to receive a mating portion of the fluid reservoir 203 (e.g., via a threaded engagement, a snap fit, tabs, etc.), and an upper (downstream) portion 220b defining a channel 228 and configured to receive an upstream end of the tubing 210. The connector 214 may further include a coupling element 224 configured to be received in the channel 228, a needle 222 having an upstream end configured to penetrate a septum of the fluid reservoir 203, and a filter 206 comprising first, second, and third filters 206a, 206b, and 206c. The filters 206 are positioned within the channel 228 with the first and second filters 206a, 206b upstream of the third filter 206c. As can be seen in FIG. 4A, each of the first and second filters 206a, 206b have a height and volume greater than a height and volume of the third filter 206c. The first and second filters 206a, 206b can comprise a porous structure formed of a first hydrophilic material having a first pore size, and the third filter 206c can comprise a porous structure formed of a second hydrophilic material different than the first hydrophilic material, and with a pore size smaller than the pore size of the first and second filters 206b, 206c. One, some, or all of the first, second, and third filters 206a, 206b, and 206c can include a hydrophilic additive, such as glycerol, as detailed above.

As best shown in FIG. 4B, the needle 222 can be secured within the body 220 such that an upstream end of the needle 222 is positioned within an interior cavity of the lower portion 220a, and a downstream end of the needle 222 is positioned within the channel 228, upstream of or aligned with an upstream end of the first filter 206a. During a fluid delivery operation, the fluid medication is forced out of the fluid reservoir 203 into the hollow needle 222, then sequentially through the first, second, and third filters 206a, 206b, 206c before flowing into the upstream end of the tubing 210 coupled to the upper portion 220b of the body 220.

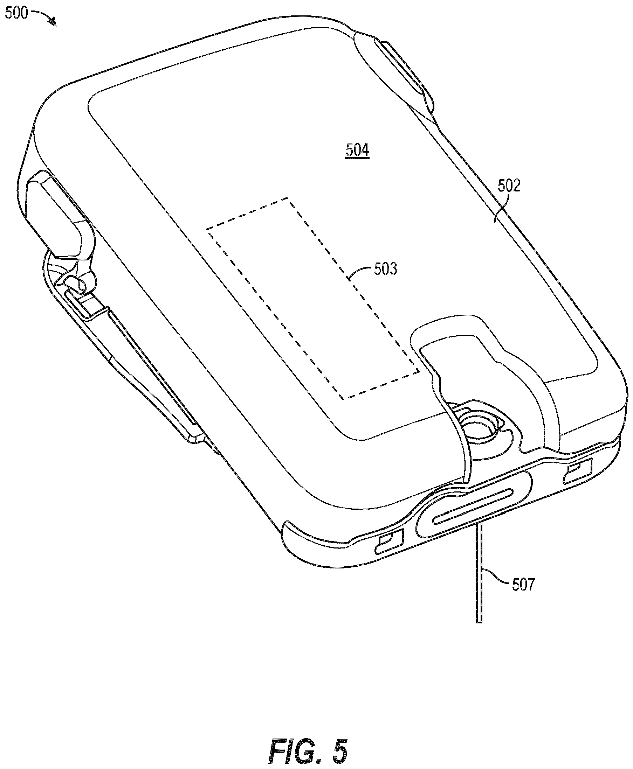

FIG. 5 is a perspective view of another embodiment of a fluid delivery system 500 that comprises a fluid infusion device 502 configured to be adhered to the skin of the user (e.g., via an adhesive patch) and deliver a fluid medication to a user via a cannula 507 associated with the infusion device 502. The fluid infusion device 502 further includes: a fluid reservoir 503 (hidden from view in FIG. 5), a plunger slidably disposed within the fluid reservoir 503, a drive system for moving the plunger to dispense the fluid medication from the fluid reservoir 503 into the cannula 507, a power source, one or more electronic components (e.g., a processor, a transmitter, etc.), and an insertion device for inserting the cannula 507 into the body of the patient. All of the foregoing components may be partially or completely contained within the housing 404.

The infusion device 502 can further include one or more filters (not visible) of the present technology disposed at one or more locations along the fluid flow path. For example, the infusion device 502 can include a filter positioned within the reservoir and/or within an internal structure of the infusion device 502 defining the portion of the fluid path between the fluid reservoir 503 and the cannula 507. Other locations are possible. The filter(s) included within the infusion device 502 can include a hydrophilic additive, such as glycerol as detailed above.

### Example

The present technology is further illustrated by the following non-limiting example.

The inventors conducted an animal study to assess the performance of different filters within a reservoir-to-tube connector (or "RT Connector") of a fluid delivery system, such as the tethered delivery system shown above in FIG. 2. Each of the tested fluid delivery systems was placed on the abdomen of a pig, with a cannula of the system inserted into a subcutaneous space of the pig. The delivery systems operated continuously to deliver insulin (via a Medtronic MiniMed^{™} 770G pump) until system failure or for 1 week (≥7 days). The dosing scheme included adjusting basal/bolus rates to control blood glucose (BG) in the range of 100-400 mg/dL. The time to system failure was determined based on: (1) the BG level not decreasing by at least 50 mg/dL an hour after a correction bolus for a BG >400 mg/dL in pigs (in humans the limit is normally set at 250-300 mg/dL), or (2) evidence of infection at the infusion site. Reasons for each delivery system failure were analyzed, including pump/fluid path occlusions due to cannula kinking, other mechanical issues, accidental device detachment/dislodgment due to animal rubbing/other movements, or infusion site loss due to insulin absorption rate decrease with time.

The control group for the study utilized RT connectors with a PVA filter that had been cut to size with a die. The control filter did not include a glycerol additive.

The study included two test products, each comprising a modified form of the control group product. The first group of test products utilized the same RT connector as the control connector, but modified to include a PVA filter that had been laser cut to size. The first test group did not include a glycerol additive. The second group of test products utilized the same RT connector as the control connector, but modified to include a PVA filter that had been laser cut to size and included a glycerol additive.

The RT connector in the control group and both RT connectors in the test groups were fluidly coupled to the same pump model (Medtronic MiniMed^{™} 770G pump) once installed on the pig.

A Kaplan-Meier plot with log rank statistics was used to assess survival rates of the control and test delivery systems. The survival rates for delivery systems with system failure due to all causes (including site loss, mechanical issue or accidental cannula pull out) are shown in FIG. 6

The summary results for the survival rates of the control and first and second test groups are shown in FIG. 7. The 7-day survival rate of the first test group (laser-cut without glycerol) was 55% and trended lower than that for the second test group (laser-cut with glycerol) (82%) and the control (die-cut without glycerol control) (78%). Thus, between the laser-cut test products, the addition of glycerol to the filter improved the survival rate.

### Conclusion

Several other embodiments of the technology can have different configurations, components, or procedures than those described herein. A person of ordinary skill in the art, therefore, will accordingly understand that the technology can have other embodiments with additional elements, or the technology can have other embodiments without several of the features shown and described above with reference to FIGS. 1-7.

The descriptions of embodiments of the technology are not intended to be exhaustive or to limit the technology to the precise form disclosed above. Where the context permits, singular or plural terms may also include the plural or singular term, respectively. Although specific embodiments of, and examples for, the technology are described above for illustrative purposes, various equivalent modifications are possible within the scope of the technology, as those skilled in the relevant art will recognize. For example, while steps are presented in a given order, alternative embodiments may perform steps in a different order. The various embodiments described herein may also be combined to provide further embodiments.

As used herein, the terms "generally," "substantially," "about," and similar terms are used as terms of approximation and not as terms of degree, and are intended to account for the inherent variations in measured or calculated values that would be recognized by those of ordinary skill in the art.

Moreover, unless the word "or" is expressly limited to mean only a single item exclusive from the other items in reference to a list of two or more items, then the use of "or" in such a list is to be interpreted as including (a) any single item in the list, (b) all of the items in the list, or (c) any combination of the items in the list. Additionally, the term "comprising" is used throughout to mean including at least the recited feature(s) such that any greater number of the same feature and/or additional types of other features are not precluded. It will also be appreciated that specific embodiments have been described herein for purposes of illustration, but that various modifications may be made without deviating from the technology. Further, while advantages associated with certain embodiments of the technology have been described in the context of those embodiments, other embodiments may also exhibit such advantages, and not all embodiments need necessarily exhibit such advantages to fall within the scope of the technology. Accordingly, the disclosure and associated technology can encompass other embodiments not expressly shown or described herein.

Further disclosed herein is the subject-matter of the following clauses:
1. A system for delivering a fluid medication to a patient at an infusion site, the system comprising:
   a fluid conduit configured to transport a fluid medication along at least a portion of a fluid path extending between a reservoir containing the fluid medication and a cannula subcutaneously inserted in a patient at an infusion site; and
   a filter disposed within the fluid conduit and configured to trap particulates that form in the fluid medication, wherein the filter comprises a porous structure including glycerin.
2. The system of clause 1, wherein the fluid medication is insulin, and wherein the filter is configured to trap insulin aggregates that form in the insulin.
3. The system of clause 1 or clause 2, wherein the system is configured to infuse the fluid medication to the patient at the infusion site over a period of time greater than four days.
4. The system of any one of clauses 1 to 3, wherein the porous structure comprises polyvinyl alcohol, a cellulose, a polyurethane, a polyester, a polyether, or a collagen.
5. The system of any one of clauses 1 to 4, wherein the porous structure has an average pore size of from about 0.1 mm to about 0.5 mm.
6. The system of any one of clauses 1 to 5, wherein the porous structure comprises at least one of an acrylic copolymer membrane, a polyethersulfone membrane, a mixed cellulose esters membrane, a cellulose acetate membrane, a cellulose nitrate membrane, a nylon membrane, a hydrophilic polytetrafluoroethylene (PTFE) membrane, and/or a polycarbonate membrane.
7. The system of any one of clauses 1 to 6, wherein the porous structure has an average pore size of from about 0.1 µm to about 10 µm.
8. The system of any one of clauses 1 to 7, wherein the fluid conduit comprises tubing and a connector configured to fluidly couple the tubing to the reservoir, and wherein the filter is disposed within the connector.
9. The system of any one of clauses 1 to 8, wherein the filter is a first filter, and wherein the system includes a second filter disposed within the fluid conduit.
10. A system for delivering a fluid medication to a patient at an infusion site, the system comprising:
   a fluid conduit having a first end configured to be fluidly coupled to a reservoir containing the fluid medication and a second end configured to be fluidly coupled to a cannula, wherein the fluid conduit is configured to transport the fluid medication from the reservoir to the patient through the cannula;
   a connector configured to fluidly couple the first end of the fluid conduit to the reservoir; and
   a filter disposed within the connector and configured to trap particulates that form in the fluid medication, the filter comprising a porous structure including glycerin.
11. The system of clause 10, wherein the fluid medication is insulin, and wherein the filter is configured to trap insulin aggregates that form in the insulin.
12. The system of clause 10 or clause 11, wherein the system is configured to infuse the fluid medication to the patient at the infusion site over a period of time greater than four days.
13. The system of any one of clauses 10 to 12, wherein the porous structure comprises polyvinyl alcohol, a cellulose, a polyurethane, a polyester, a polyether, or a collagen.
14. The system of any one of clauses 10 to 13, wherein the porous structure has an average pore size of from about 0.1 mm to about 0.5 mm.
15. The system of any one of clauses 10 to 14, wherein the porous structure comprises at least one of an acrylic copolymer membrane, a polyethersulfone membrane, a mixed cellulose esters membrane, a cellulose acetate membrane, a cellulose nitrate membrane, a nylon membrane, a hydrophilic polytetrafluoroethylene (PTFE) membrane, and/or a polycarbonate membrane
16. The system of any one of clauses 10 to 15, wherein the porous structure has an average pore size of from about 0.1 µm to about 10 µm.
17. The system of any one of clauses 10 to 16, wherein the fluid conduit comprises tubing.
18. The system of any one of clauses 10 to 17, wherein the filter is a first filter, and wherein the system includes a second filter disposed within the fluid conduit.
19. A system for delivering a fluid medication to a patient at an infusion site, the system comprising:
   an infusion pump;
   a reservoir configured to store the fluid medication, the reservoir configured to be received by the infusion pump;
   a cannula configured for subcutaneous insertion into a tissue of the patient at the infusion site;
   a fluid conduit having a first end configured to be fluidly coupled to the reservoir and a second end configured to be fluidly coupled to the cannula, wherein the fluid conduit is configured to transport the fluid medication from the reservoir to the patient; and
   a connector configured to fluidly couple the first end of the fluid conduit to the reservoir; and
   one or more filters disposed in at least one of the reservoir, the fluid conduit, or the connector, wherein each of the filters is configured to trap particulates that form in the fluid medication, and wherein the filter comprises a porous structure including glycerin.
20. The system of clause 19, wherein the fluid medication is insulin, and wherein the filter is configured to trap insulin aggregates that form in the insulin.
21. A system for delivering a fluid medication to a patient at an infusion site, the system comprising:
   a fluid conduit having a first end configured to be fluidly coupled to a reservoir containing the fluid medication and a second end configured to be fluidly coupled to a cannula, wherein the fluid conduit is configured to transport the fluid medication from the reservoir to the patient through the cannula;
   a connector configured to fluidly couple the first end of the fluid conduit to the reservoir; and
   a filter disposed within the connector and configured to trap particulates that form in the fluid medication, the filter comprising a porous structure having an absorption rate between 0 and 30 seconds.
22. A system for delivering a fluid medication to a patient at an infusion site, the system comprising:
   a fluid conduit configured to transport a fluid medication along at least a portion of a fluid path extending between a reservoir containing the fluid medication and a cannula subcutaneously inserted in a patient at an infusion site; and
   a filter disposed within the fluid conduit and configured to trap particulates that form in the fluid medication, wherein the filter comprises a porous structure having an absorption rate between 0 and 30 seconds.
23. A system for delivering a fluid medication to a patient at an infusion site, the system comprising:
   an infusion pump;
   a reservoir configured to store the fluid medication, the reservoir configured to be received by the infusion pump;
   a cannula configured for subcutaneous insertion into a tissue of the patient at the infusion site;
   a fluid conduit having a first end configured to be fluidly coupled to the reservoir and a second end configured to be fluidly coupled to the cannula, wherein the fluid conduit is configured to transport the fluid medication from the reservoir to the patient; and
   a connector configured to fluidly couple the first end of the fluid conduit to the reservoir; and
   one or more filters disposed in at least one of the reservoir, the fluid conduit, or the connector, wherein each of the filters is configured to trap particulates that form in the fluid medication, and wherein the filter comprises a porous structure having an absorption rate between 0 and 30 seconds.

## Claims

1. A system for delivering a fluid medication to a patient at an infusion site, the system comprising:
a fluid conduit configured to transport a fluid medication along at least a portion of a fluid path extending between a reservoir containing the fluid medication and a cannula subcutaneously inserted in a patient at an infusion site; and
a filter disposed within the fluid conduit and configured to trap particulates that form in the fluid medication, wherein the filter comprises a porous structure including glycerin.

2. The system of Claim 1, wherein the fluid medication is insulin, and wherein the filter is configured to trap insulin aggregates that form in the insulin.

3. The system of Claim 1 or Claim 2, wherein the system is configured to infuse the fluid medication to the patient at the infusion site over a period of time greater than four days.

4. The system of any one of Claims 1 to 3, wherein the porous structure comprises polyvinyl alcohol, a cellulose, a polyurethane, a polyester, a polyether, or a collagen.

5. The system of any one of Claims 1 to 4, wherein the porous structure has an average pore size of from about 0.1 mm to about 0.5 mm.

6. The system of any one of Claims 1 to 5, wherein the porous structure comprises at least one of an acrylic copolymer membrane, a polyethersulfone membrane, a mixed cellulose esters membrane, a cellulose acetate membrane, a cellulose nitrate membrane, a nylon membrane, a hydrophilic polytetrafluoroethylene (PTFE) membrane, and/or a polycarbonate membrane.

7. The system of any one of Claims 1 to 6, wherein the porous structure has an average pore size of from about 0.1 µm to about 10 µm.

8. The system of any one of Claims 1 to 7, wherein the fluid conduit comprises tubing and a connector configured to fluidly couple the tubing to the reservoir, and wherein the filter is disposed within the connector.

9. The system of any one of Claims 1 to 8, wherein the filter is a first filter, and wherein the system includes a second filter disposed within the fluid conduit.
